# EUROPÄISCHE PATENTSCHRIFT

(11) **EP 1 469 804 B1**
(45) Veröffentlichungstag und Bekanntmachung des Hinweises auf die Patenterteilung: **08.08.2007**
(21) Anmeldenummer: 03704237.1
(22) Anmeldetag: 22.01.2003
(51) Int. Cl.: A61F 5/01

(54) **SAUGGLOCKE ZUR NICHT-CHIRURGISCHEN KORREKTUR VON FORM UND/ODER FUNKTIONSLEISTUNG DES BRUSTKASTENS**
SUCTION CUP FOR NON-SURGICAL CORRECTION OF THE FORM AND/OR FUNCTIONALITY OF THE CHEST
CLOCHE DE SUCCION SERVANT A CORRIGER DE MANIERE NON CHIRURGICALE LA FORME ET/OU LA CAPACITE DE FONCTIONNEMENT DU THORAX

(30) Priorität: 01.02.2002 DE 10204078
(43) Veröffentlichungstag der Anmeldung: 27.10.2004
(73) Patentinhaber: Klobe, Eckart, 68161 Mannheim (DE)
(72) Erfinder: Klobe, Eckart, 68161 Mannheim (DE)
(74) Vertreter: Meyer-Roedern, Giso
(86) Internationale Anmeldenummer: PCT/DE2003/000161
(87) Internationale Veröffentlichungsnummer: WO 2003/063752

(56) Entgegenhaltungen:
- EP-A- 0 338 524
- DE-A- 2 450 098
- DE-A- 19 734 571
- DE-U- 9 400 822
- US-A- 6 098 205

## Beschreibung

Die Erfindung betrifft eine Saugglocke zur nicht-chirurgischen Korrektur von Form und/oder Funktionsleistung des Brustkastens, die an der Vorderseite des Rumpfes in abdichtende Anlage kommt, und mit der an den Körper Unterdruck angelegt werden kann.

Unterdruck ist zu verstehen als der Betrag einer auf den Atmosphärendruck bezogenen Druckdifferenz.

Bei der Erfindung geht es darum, Knochen und Knorpel des Brustkastens in ihrer anatomischen Konfiguration zu beeinflussen und speziell ein Biegemoment auf die knöchernen und knorpeligen Bestandteile der Rippen, des Brustbeins und der Rippenbögen auszuüben, und zwar in erster Linie zur nicht-chirurgischen Korrektur der Trichterbrust. Mit der verschönernden Behandlung von Weichteilen oder deren Rekonstruktion ist die Erfindung nicht oder allenfalls in zweiter Linie befaßt.

In der medizinischen Fachwelt gilt die chirurgische Operation als Mittel der Wahl zur effektiven Korrektur der Trichterbrust (vgl. Morphologische und biochemische Untersuchungen in Trichter- und Kielbrustdeformierungen unter Berücksichtigung der nicht kollagenen Knorpelkomponenten, Sonja Nitsche, Medizinische Fakultät der Westfälischen Wilhelmsuniversität Münster, Dissertation 2000, Seiten 19 bis 25). Konservative Maßnahmen - krankengymnastische Übungen und orthopädietechnische Maßnahmen - werden in dieser Quelle als nicht in der Lage beschrieben, den Trichter zu beseitigen.

Nach dieser Quelle gibt es rein kosmetische Operationen, welche die optische Erscheinung des Trichters kaschieren. Dies geschieht etwa durch Einlagerung von geeignetem Material unter die Haut oder dadurch, daß Rippen von dem den Trichtergrund bildenden Brustbeins gelöst, über das Brustbein zur Gegenseite gezogen und auf der Gegenseite fixiert werden. Die Auswirkungen des Trichters auf die inneren Organe werden dadurch jedoch nicht beseitigt. Andere Operationen bestehen darin, die Verbindung zwischen dem Brustbeinkörper und dem Schwertfortsatz zu durchtrennen und dann das darunterliegende Zwerchfell vom Brustbein so loszuschneiden, daß es beim Einatmen keine weiteren nach innen gerichteten Zugkräfte mehr auf das Brustbein bewirken kann. Derartige Operationen gelten als Zwischenlösung, mit denen sogenannte Radikaloperationen aufgeschoben werden können. Zu den Radikaloperationen gehören solche von Entfernung des Brustbeins bis zur Kombination aus Mobilisierung des Brustbeins und Fixierung in überkorrigierter Stellung. Eine der üblichen Radikaloperationen erfordert einen Hautschnitt von der Drosselgrube bis zum Nabel, Abtrennung der auf dem Brustkorb sitzenden Muskulatur, Durchtrennung der Ursprünge des Bauchmuskels in Höhe des Schwertfortsatzes, Einschnitte in die Rippenknorpelhaut, vorsichtiges Abschieben der Knorpelhaut, Knorpeldurchtrennung, Durchschlag des Brustbeins und Fixierung desselben mittels eines Metallbügels, der gegebenenfalls durch zwei weitere Bügel ergänzt wird, wobei die Enden der Bügel durch Schränkeisen so verbogen werden, daß sie sich der Thoraxapertur anpassen und das darüberliegende Gewebe nicht anheben, Fixierung der Bügel, gegebenenfalls Abschrägung abstehender Rippenansätze, Anlage einer Redondrainage, Verschließen der Wunde, 10 bis 14 Tage stationärer Krankenhausaufenthalt, intensive atemtherapeutische Unterstützungsmaßnahmen und Verbleib der Metallbügel für zwölf Monate an eingebauter Stelle.

An anderer Stelle (vgl. Die Trichterbrust. Stadien- und formgerechte Korrektur, Dr. Hans Peter Hümmer, Zuckschwerdt Verlag München, Bern, Wien, 1985, Seiten 10, 11, 26, 31 und 32) findet sich die Einteilung der Trichterbrustkorrektur in drei Phasen - Mobilisierung, Stabilisierung, Weichteilrekonstruktion - und der Hinweis, daß historisch gesehen der Erfolg der Trichterbrustoperationen zunächst durch mangelhafte Stabilisierungsmöglichkeiten beeinträchtigt wurde. Dieselbe Quelle beschreibt unter anderem auch den Einsatz von Drähten, Nägeln, Spangen oder Stahlbügeln als Hilfsmittel, die Monate oder Jahre im Körper verbleiben oder aus dem Körper herausragen, um dem Brustkorb nach erfolgter Trichterbrustoperation (Mobilisierung des Brustbeins) die nötige Stabilität zu verleihen. Dieselbe Quelle erwähnt zwar Alternativen einer nicht-chirurgischen Korrektur der Trichterbrust, beschreibt sie aber als ineffektiv, speziell auch eine Korrektur mittels Saugglocke basierend auf der nachstehend behandelten Literaturstelle Spitzy.

Bei Spitzy (Handbuch der Kinderheilkunde, Band 8, Othopädie im Kindesalter, Prof. Dr. Hans Spitzy, Verlag von F.C.W. Vogel in Leipzig, 3. Auflage, 1930, Seiten 196 und 197) wird beschrieben, daß durch den Einsatz einer Glasglocke über der eingesunkenen Partie einer Trichterbrust unter Einwirkung von Unterdruck eine sofortige Hebung des Trichters feststellbar war. Jedoch wurde die Technik der Methode als schwierig beschrieben, weil es schwer war, die starre Wand der dort verwendeten Saugglocke den unebenen Flächen der vorderen Thoraxwand anzupassen.

Aus der DE 197 34 571 A1 ist zur Behandlung der Trichterbrust eine Saugglocke mit einer zentralen, transparenten Scheibe und einem individuell auf den jeweiligen Patienten abgestimmten oder abstimmbaren Rand bekannt, der auf dessen Körper weich aufliegt. Mit der Saugglocke sollen ein- bis zweimal pro-Woche Therapie-Einheiten von 5 bis 15 min durchgeführt werden.

Nachteile des bisherigen Stands der Technik bestehen bei konservativen Verfahren in einem tief verwurzelten und weit verbreiteten Vorurteil zu Lasten diesbezüglicher Möglichkeiten; bei den kosmetischen Eingriffen in einer rein kosmetischen Wirkung, welche die Auswirkungen des Trichters auf die inneren Organe nicht beseitigt; bei der Abtrennung des Zwerchfells vom Brustbein in einer rein hinhaltenden Wirkung; und bei den Radikaloperationen in der Schwere der Belastung durch die Operation und die über Monate oder Jahre implantiert bleibenden Metallkörper zur Fixierung der operierten Trichterbrust - ganz abgesehen von den teils erheblichen nachoperativen Schmerzen bei den Patienten -; bei den Saugglocken nach Spitzy im Fehlen jeglichen Hinweises auf eine erfolgreiche Stabilisierung in korrigiertem Zustand, und bei der DE 197 34 571 A1 in der Konzipierung auf kurze Anwendungseinheiten.

Aus der DE 42 28 406 C2 ist eine Schröpfvorrichtung mit einer Saugglocke bekannt.

Aufgabe der Erfindung ist es, eine Saugglocke der eingangs genannten Art soweit alltagstauglich zu machen, daß sie den nachstehenden Kriterien bestmöglich genügt:
1. Die Saugglocke soll in der Lage sein, neben einer Mobilisierung des Brustbeins auch eine ausreichende Stabilisierung des Brustbeins in korrigierter Lage zu gewährleisten. Hierzu soll sie auch außerhalb therapeutischer Einrichtungen über weite Teile des Tages und/oder der Nacht angewandt werden können, ohne daß sie der Ausführung allgemein üblicher Körperbewegungen und/oder Tätigkeiten ernsthaft im Wege steht. Das betrifft insbesondere das Liegen, Sitzen, Stehen, Gehen, Laufen, Schulbesuch, Büroarbeit, Hausarbeit, und verschiedene Arten von körperlicher Arbeit oder Sport. Hierzu soll die Saugglocke angenehm zu tragen sein und eine gute Abdichtung gewährleisten.
2. Es soll eine Selbstbedienung der Saugglocke durch den Patienten mit nur gelegentlicher ärztlicher Kontrolle möglich sein. Hierzu soll die Saugglocke eine Reihe von Sicherheitsfunktionen gewährleisten.
3. Die Saugglocke soll hinsichtlich ihrer Wirkung eine hohe Funktionalität aufweisen und sich den anatomischen Besonderheiten verschiedener Anwender flexibel anpassen, ohne irgendwelche Umbauten zu benötigen. Sie soll ihre Haupteinwirkung an den Orten der maximalen therapeutischen Wirksamkeit entfalten. Bei durch Behandlungsfortschritt sich ändernden Orten maximaler therapeutischer Wirksamkeit soll sie in der Lage sein, mit ihrer Haupteinwirkung diesen Orten zu folgen.
4. Durch ihre häufigere Anwendung und die jeweils längere Einwirkzeit der Saugglocke soll ihre therapeutische Wirksamkeit verbessert und ein schnellerer sowie nachhaltigerer Behandlungserfolg erzielt werden. Insbesondere soll sehr rasch der Zeitpunkt erreicht werden, an dem der durch die Saugglocke angehobene Trichter in den Anwendungspausen zwischen den Einzelanwendungen nicht mehr in die alte Position zurückfällt, was sich auf die psychische Befindlichkeit des Patienten und seine Bereitschaft zur fortgesetzten Anwendung positiv auswirkt.

Die diese Aufgabe lösende Saugglocke besteht wenigstens zum Teil aus weichelastischem Material. Sie weist unter Atmosphärendruck zum Körper hin sich erweiternde innere Flanken auf. Die Saugglocke liegt mit seitlichen Körperanlagepartien seitlich des Brustbeins, mit einer oberen Körperanlagepartie im oberen Bereich des Brustbeins und mit einer unteren Körperanlagepartie im unteren Bereich oder unterhalb des Brustbeins an. Die Verformbarkeit der Saugglocke variiert über ihren Zentriwinkel. Die Verformbarkeit zumindest zwischen der oberen, der unteren oder einer der seitlichen Körperanlagepartie(n) ist verschieden.

Mit zunehmendem Unterdruck erfährt die Saugglocke eine Verformung, durch die sich ihre Berührfläche mit dem Körper zunehmend vergrößert, und durch die sich die von ihr überwölbte Fläche zunehmend verringert.

Mit der erfindungsgemäßen Saugglocke gehen folgende Vorteile einher:
1. Die bei zunehmendem Unterdruck auftretende Verringerung der von der Saugglocke überwölbten Fläche bewirkt zweierlei: Gesamt-Saugkräfte und damit Gesamt-Auflagekräfte, die unterproportional zur Zunahme des Unterdrucks ausfallen, sowie eine zunehmende Eingrenzung derjenigen Flächen, die den zunehmenden Unterdrucken weiterhin ausgesetzt sind. Dies dient der Sicherheit, dem Tragekomfort und gestattet eine lokal unterschiedliche Schonung saugkraftempfindlicher Körperteile.
2. Die zum Körper hin sich erweiternden inneren Flanken, die bei Zunahme des Unterdrucks ihre Berührfläche mit dem Körper zunehmend vergrößern, bewirken bei Zunahme der Saugkräfte erstens eine zunehmende Verringerung derjenigen Entfernungen, die ein Teil des Körpers in Richtung des Innengewölbes der Saugglocke zurücklegen könnte, bevor er dort anstieße und zweitens erlauben sie - sofern sie selbst aus weichelastischem Material bestehen - zunehmend eigenmotorische Bewegungen des Körpers, ohne daß die Saugglocke dabei an Saughaftung verliert. Dies bietet zum ersten eine Sicherungsfunktion gegenüber ausdehnungsempfindlichen Körperteilen, und zum zweiten erlaubt es die Ausübung eigener Körperbewegungen, ohne dadurch die therapeutische Einwirkung der Saugglocke zu unterbrechen.
3. Durch die zum Körper hin sich erweiternden inneren Flanken lassen sich die Bereiche maximaler Biegemomentsausübung dorthin bringen, wo bei gängigen chirurgischen Korrekturen zur Mobilisierung des Trichters die Knochen und Knorpel zerschnitten oder angeschnitten werden: in den Bereich des Trichterbrustrands und in den Bereich des Brustbeins. Und sollte - etwa durch Behandlungsfortschritt - die Trichterform sich ändern, so folgt der Bereich der maximalen Biegemomentsausübung dem jeweiligen Trichterbrustrand und damit dem Bereich der therapeutisch wirksamsten Biegemomentsausübung.

Dadurch, daß die Verformbarkeit der Saugglocke über ihren Zentriwinkel variiert, variiert auch die bei anliegendem Unterdruck sich ergebende Flächenpressung der Saugglocke über ihren Zentriwinkel.

Der Begriff Verformbarkeit kann beinhalten Verformungswiderstand gegenüber der Erzeugung eines Verformungswegs, Rückstellkraft nach der Erzeugung eines Verformungswegs und Verformungsweg bei Erfahrung einer Krafteinwirkung. Getestet werden kann die Verformbarkeit mit der Stirnfläche eines zylindrischen Prüfkörpers jeweils flächennormal zu den inneren Flanken der Saugglocke, wobei der Zylinderradius 8 mm betragen kann.

Die über den Zentriwinkel variierende Verformbarkeit der Saugglocke ermöglicht es, den vorgenannten Vorteilen bevorzugte Sektoren zuzuweisen. Dies betrifft insbesondere Sektoren mit geringer Druckkraftausübung zur Schonung druckkraftempfindlicher Bereiche, Sektoren mit geringer Saugkraftausübung zur Schonung saugkraftempfindlicher Bereiche, Sektoren mit engerer Ausdehnungsbegrenzung zur Schonung ausdehnungsempfindlicher Bereiche, Sektoren erhöhter Toleranz gegenüber eigenmotorischen Bewegungen zur Erhöhung des Bewegungskomforts, und Sektoren erhöhter oder geringerer Biegemomentsausübung zur Behandlung von Bereichen größeren bzw. geringeren Einwirkungsbedarfs sowie zur Vorgabe von Vorzugsrichtungen für die auszuübenden Biegemomente.

Bei einer bevorzugten Ausführungsform liegt die Saugglocke mit schwer verformbaren seitlichen Körperanlagepartien seitlich des Brustbeins, mit einer mittelschwer verformbaren oberen Körperanlagepartie im oberen Bereich des Brustbeins und mit einer leicht verformbaren unteren Körperanlagepartie im unteren Bereich oder unterhalb des Brustbeins an. Der im wesentlichen muskelgestützte, druckempfindliche Körperbereich unter den Rippenbögen wird so mit weniger Flächenpressung beaufschlagt als der unempfindlichere Körperbereich im oberen Bereich des Brustbeins. Die stärkste Flächenpressung wirkt an den Rippenknochen (Os costale) und den diese bis zum Brustbein verlängernden Knorpelbrücken (Cartilago costale), so daß die Hauptbiegeachse in der Vertikalen verläuft.

Bei einer bevorzugten Ausführungsform ist das Material der Körperanlagepartien homogen und ihre unterschiedliche Verformbarkeit durch örtlich variierende Dicke gegeben. Wohlgemerkt besteht aber auch die Möglichkeit, die unterschiedliche Verformbarkeit der Körperanlagepartien durch eine Variation ihrer Materialeigenschaften zu erreichen oder eine Variation der Materialeigenschaften mit einer Dickenvariation zu kombinieren.

Bei einer bevorzugten Ausführungsform sind die seitlichen Körperanlagepartien der Saugglocke am dicksten. Ihre obere Körperanlagepartie ist dünner, und ihre untere Körperanlagepartie am dünnsten.

Bei einer bevorzugten Ausführungsform bestehen die Körperanlagepartien der Saugglocke aus Silikon. Bei der hier verwendeten, allgemein gebräuchlichen Bezeichnung "Silikon" handelt es sich chemisch um Polyorganosiloxan. Dieses Material zeichnet sich durch eine sehr gute elastische Verformbarkeit und Hautverträglichkeit aus. Die gute Hautverträglichkeit beruht im wesentlichen auf einer guten Durchlässigkeit für Sauerstoff, Stickstoff, Kohlendioxid und Wasserdampf. Das Silikon läßt sich auf unaufwendige Weise in einem abformenden Verfahren, insbesondere Gieß- oder Spritzgießverfahren, verarbeiten. Das Silikon ist bevorzugt additionsvernetzend, kann aber auch kondensationsvernetzend oder radikalisch vernetzend sein. Die Verarbeitung erfolgt vorzugsweise bei Zimmertemperatur, kann aber gewünschtenfalls durch Temperaturerhöhung oder Lichtpolymerisation beschleunigt werden.

Bei einer bevorzugten Ausführungsform haben die Körperanlagepartien der Saugglocke eine Härte zwischen 1 Shore A und 35 Shore A.

Bei einer bevorzugten Ausführungsform besteht die Saugglocke aus einem im wesentlichen biegesteifen Mittelteil und einer unter Abdichtung daran ansetzenden Schürze aus weichelastischem Material, insbesondere Silikon (Polyorga-nosiloxan).

Bei einer bevorzugten Ausführungsform ist das Mittelteil von im wesentlichen kreisrundem, kreissegmentförmigem oder kreissektorförmigem Grundriß.

Bei einer bevorzugten Ausführungsform hat die Saugglocke im Grundriß ihres Mittelteils seitliche Einbuchtungen mit im wesentlichen teilkreisförmiger Randkontur. Die Einbuchtungen dienen zur Aussparung der weiblichen Brüste.

Bei einer bevorzugten Ausführungsform erweitert sich die Schürze von dem Mittelteil nach außen. Sie hat dabei mit den seitlichen Körperanlagepartien am wenigsten, mit der oberen Körperanlagepartie mehr und mit der unteren Körperanlagepartie am meisten Auslage von dem Mittelteil.

Bei einer bevorzugten Ausführungsform verjüngt sich die Schürze ringsum mehr oder weniger von dem Mittelteil weg.

Bei einer bevorzugten Ausführungsform ist der Querschnitt der Schürze im wesentlichen dreieckig oder trapezförmig. Die Schürze kann aber auch konkav oder konvex gewölbt sein.

Bei einer bevorzugten Ausführungsform ist das Mittelteil eben. Die Schürze hat einen Saum, der in einer zu der Ebene des Mittelteils parallelen Ebene liegt. Der Saum kann aber auch konkav oder konvex gewölbt sein.

Für das Mittelteil kommt ebenfalls eine konvexe Wölbung, und zwar vorzugsweise leichte konvexe Wölbung in Betracht.

Bei einer bevorzugten Ausführungsform ist die Schürze der Saugglocke in einem abformenden Verfahren, insbesondere gieß- oder spritzgießtechnisch hergestellt. Das Mittelteil ist in die Schürze eingebettet.

Bei einer bevorzugten Ausführungsform ist zumindest die nicht von der Schürze bedeckte Partie des Mittelteils transparent. Der Patient kann dadurch den Fortschritt der Behandlung unmittelbar visuell verfolgen, was für seine Motivation von beträchtlicher Bedeutung ist.

Bei einer bevorzugten Ausführungsform ist das Mittelteil mit einem Anschluß für das Anlegen von Unterdruck versehen.

Bei einer bevorzugten Ausführungsform ist der an die Saugglocke anlegbare Unterdruck konstruktiv begrenzt. Das ist gleichbedeutend mit einer konstruktiven Begrenzung der Flächenpressung, die mit der Saugglocke maximal ausgeübt werden kann. Das ist ein wichtiges Sicherheitsmerkmal, speziell wenn die Anwendung der Saugglocke in Händen eines medizinischen Laien liegt. Die Begrenzung des Unterdrucks kann konstruktiv auf verschiedenste Art und Weise gelöst werden. Beispielsweise kann die Saugglocke in ihrer Verformbarkeit so gestaltet sein, daß sie bei Überschreiten eines vorgegebenen Grenzunterdrucks gänzlich kollabiert. Es besteht auch die Möglichkeit, die Saugglocke mit einem druckabhängig schaltenden Sicherheitsventil zu versehen. Nicht zuletzt läßt sich der Unterdruck einfach durch die Bauart einer Saugpumpe begrenzen, die an die Saugglocke angeschlossen wird.

Bei einer bevorzugten Ausführungsform ist die Saugpumpe eine Handpumpe. Sie läßt sich einfach mitführen, und sie ist von einer externen Energieversorgung unabhängig. Das ist für die Alltagstauglichkeit der Saugglocke von großer Bedeutung.

Bei einer bevorzugten Ausführungsform ist der Unterdruck, der sich mit der Handpumpe in der Saugglocke aufbauen läßt, durch die Bauart der Handpumpe konstruktiv begrenzt. Das ist ein Sicherheitsmerkmal. Erfahrungsgemäß kommt man ohne weiteres mit einem Unterdruck von ca. 0,4 Bar aus. Dieser läßt sich mit einer kleinen, unaufwendigen Handpumpe aufbauen, die ein Betätigungselement in Form eines elastisch komprimierbaren Balls hat, der ohne weiteres in eine Hemd- oder Jackettasche paßt. Wohlgemerkt kann mit einer Pumpe anderer Bauart auch ein höherer Unterdruck und eine entsprechend stärkere Krafteinwirkung erzielt werden, was aber dem medizinischen Fachpersonal vorbehalten bleiben sollte.

Die Erfindung wird im folgenden anhand von in der Zeichnung dargestellten Ausführungsbeispielen näher erläutert. Es zeigen:
Fig. 1 eine Saugglocke in Draufsicht;
Fig. 2 einen Längsschnitt durch die Saugglocke nach 11-11 von Fig. 1;
Fig. 3 einen Querschnitt durch die Saugglocke nach III-III von Fig. 1; und
Fig. 4 bis Fig. 7 die Grundrisse verschiedener Mittelteile von Saugglocken.

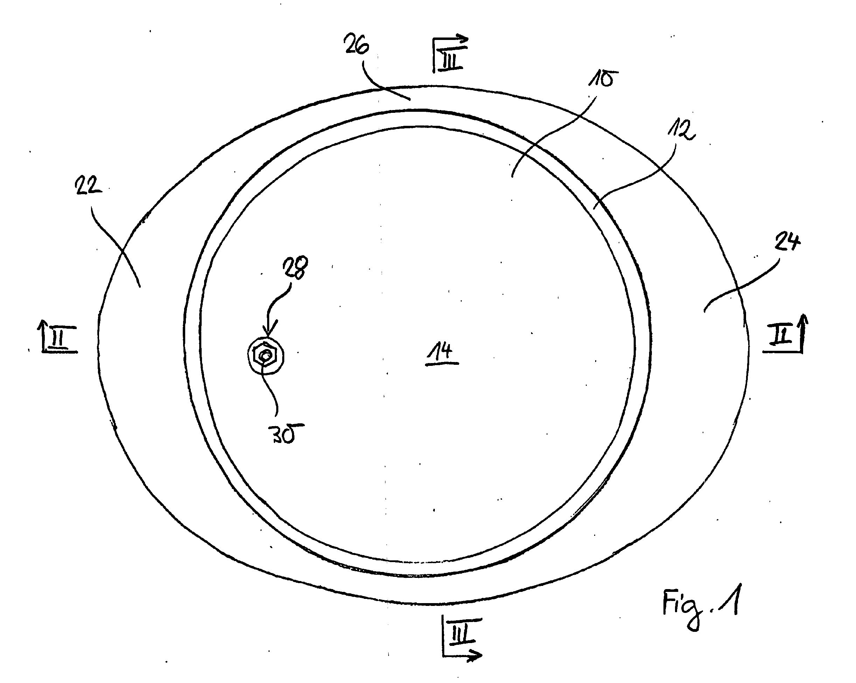

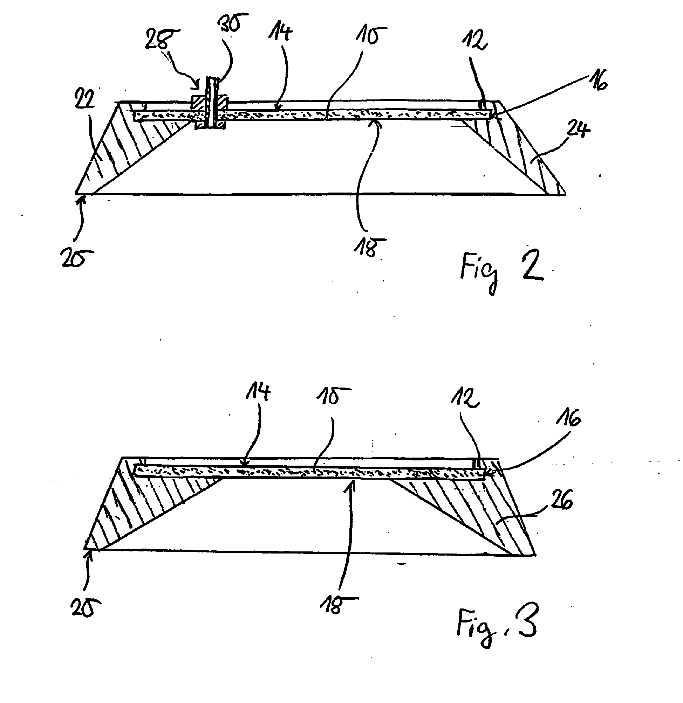

Die Saugglocke gemäß Fig. 1 bis Fig. 3 hat ein Mittelteil in Form einer kreisrunden Scheibe 10, die eben, relativ biegesteif und transparent ist. Die Scheibe 10 besteht aus splitterfreiem Kunststoff.

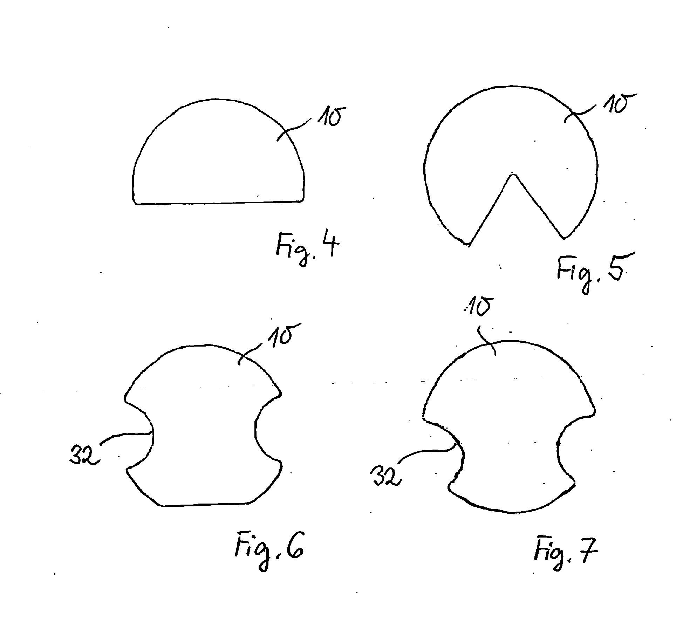

Fig. 4 bis Fig. 7 zeigen andere mögliche Grundrißformen für die Scheibe 10. Die Grundrisse gemäß Fig. 4 und Fig. 6 sind Kreissegmente. Der Grundriß gemäß Fig. 5 ist ein Kreissektor. Die Grundrisse gemäß Fig. 6 und Fig. 7 haben seitliche Einbuchtungen 32 mit teilkreisförmiger Randkontur.

Zurückkommend auf Fig. 1 bis Fig. 3, ist die Scheibe 10 an der Peripherie ringsum in eine Schürze aus weichelastischem Silikon (Polyorganosiloxan) eingefaßt. Die Schürze steht mit einem schmalen Kragen 12 von überall gleicher Dicke über die Außenseite 14 der Scheibe 10 vor, und sie überdeckt ihren seitlichen Rand 16. Die Schürze überlappt die Innenseite 18 der Scheibe 10 in einer Breite, die größer ist als die des Kragens 12. In dem Überlappungsbereich bedarf es keiner Transparenz der Scheibe 10, da die Schürze undurchsichtig ist.

Die Schürze erweitert sich von der Scheibe 10 weg. Sie hat eine konische Schräge und einen ebenen Saum 20, der in einer zu der Scheibe 10 parallelen Ebene liegt.

Der Querschnitt der Schürze ist im wesentlichen trapezförmig. Die Schürze verjüngt sich ringsum zu dem Saum 20 hin.

Die Schürze hat ein mittleres Maß an Auslage von der Scheibe 10, wo sie im oberen Brustbeinbereich des Patienten zu liegen kommt. Die Körperanlagepartie 22 der Schürze ist hier von mittlerer Dicke.

Die Schürze hat mehr Auslage von der Scheibe 10, wo sie dem oberen Brustbeinbereich gegenüber unterhalb der Rippenbögen des Patienten zu liegen kommt. Die Körperanlagepartie 24 ist hier am dünnsten.

Die Schürze hat beidseits am wenigsten Auslage von der Scheibe 10, wo sie an den Rippenknochen (Os costale) und den sie zum Brustbein hin verlängernden Knorpelbrücken (Cartilago costale) des Patienten zu liegen kommt. Ihre Körperanlagepartie 26 ist hier am dicksten.

Die Scheibe 10 ist im oberen Brustbeinbereich des Patienten mit einem Anschluß 28 versehen. Dieser hat eine Schlauchtülle 30, auf die sich ein Schlauch aufstecken läßt, der zu einer Handpumpe führt. Der Anschluß 28 kann aus Kunststoff bestehen und in die Scheibe 10 eingeklebt sein.

Zur Wirkungsweise: Bei Abwesenheit dritter Kräfte und unter Vernachlässigung des Eigengewichts der Saugglocke ist ihre Saugkraft auf denjenigen Kraftbetrag begrenzt, den die Ränder der Saugglocke in der Lage sind, vorzugsweise im Bereich des Trichterbrustrands, als Druckkraft aufzubringen. Begrenzt man konstruktiv die Tragkraft der Saugglockenränder in gezielten Bereichen, so kann man den Auflagedruck der Saugglocke in diesen Bereichen gezielt begrenzen. Entsprechend kann man auch die Ausübung der Biegemomente durch die Saugglocke konstruktiv durch die Tragkraft ihrer Ränder beeinflussen. Gegenkräfte dazu sind die Saugkraft in dem von der Saugglocke überwölbten Bereich und die Haltekraft der Rippen im Bereich der Wirbelsäule.

### Liste der Bezugszeichen

- 10: Scheibe
- 12: Kragen
- 14: Außenseite
- 16: Rand
- 18: Innenseite
- 20: Saum
- 22: obere Körperanlagepartie
- 24: untere Körperanlagepartie
- 26: seitliche Körperanlagepartie
- 28: Anschluß
- 30: Schlauchtülle
- 32: Einbuchtung

## Patentansprüche

1. Saugglocke zur nicht-chirurgischen Korrektur von Form und/oder Funktionsleistung des Brustkastens, die an der Vorderseite des Rumpfes in abdichtende Anlage kommt, und mit der an den Körper Unterdruck anlegbar ist, **dadurch gekennzeichnet, daß** die Saugglocke wenigstens zum Teil aus weichelastischem Material besteht, unter Atmosphärendruck zum Körper hin sich erweiternde innere Flanken aufweist und mit seitlichen Köperanlagepartien (26) seitlich des Brustbeins, mit einer oberen Köperanlagepartie (22) im oberen Bereich des Brustbeins und mit einer unteren Körperanlagepartie (24) im unteren Bereich oder unterhalb des Brustbeins anlegbar ist, und daß die Verformbarkeit der Saugglocke über ihren Zentriwinkel variiert und zumindest zwischen der oberen, der unteren oder einer der seitlichen Köperanlagepartie(n) (22, 24, 26) verschieden ist.

2. Saugglocke nach Anspruch 1, **dadurch gekennzeichnet, daß** die seitlichen Körperanlagepartien (26) schwer verformbar sind, daß die obere Körperanlagepartie (22) mittelschwer verformbar ist, und daß die untere Körperanlagepartie (24) leicht verformbar ist.

3. Saugglocke nach Anspruch 1 oder 2, **dadurch gekennzeichnet, daß** das Material der Körperanlagepartien (22, 24, 26) homogen und ihre unterschiedliche Verformbarkeit durch örtlich variierende Dicke gegeben ist.

4. Saugglocke nach einem der Ansprüche 1 bis 3, **dadurch gekennzeichnet, daß** die seitlichen Körperanlagepartien (26) dick sind, die obere Körperanlagepartie (22) dünner ist und die untere Körperanlagepartie (24) am dünnsten ist.

5. Saugglocke nach einem der Ansprüche 1 bis 4, **dadurch gekennzeichnet, daß** die Körperanlagepartien (22, 24, 26) aus Silikon (Polyorganosiloxan) bestehen.

6. Saugglocke nach einem der Ansprüche 1 bis 5, **dadurch gekennzeichnet, daß** die Körperanlagepartien (22, 24, 26) eine Härte zwischen 1 Shore A und 35 Shore A haben.

7. Saugglocke nach einem der Ansprüche 1 bis 6, **dadurch gekennzeichnet, daß** sie aus einem im wesentlichen biegesteifen Mittelteil und einer unter Abdichtung daran ansetzenden Schürze aus weichelastischem Material, insbesondere Silikon (Polyorganosiloxan), besteht.

8. Saugglocke nach Anspruch 7, **dadurch gekennzeichnet, daß** das Mittelteil von im wesentlichen kreisrundem, kreissegmentförmigem oder kreisektorförmigem Grundriß ist.

9. Saugglocke nach Anspruch 7 oder 8, **dadurch gekennzeichnet, daß** sie im Grundriß ihres Mittelteils seitliche Einbuchtungen (32) mit im wesentlichen teilkreisförmiger Randkontur hat.

10. Saugglocke nach einem der Ansprüche 7 bis 9, **dadurch gekennzeichnet, daß** sich die Schürze von dem Mittelteil nach außen erweitert und dabei mit den seitlichen Körperanlagepartien (26) am wenigsten, mit der oberen Körpera-nlagepart-ie (-22) mehr und mit der unteren Körperanlagepartie (24) am meisten Auslage von dem Mittelteil hat.

11. Saugglocke nach einem der Ansprüche 7 bis 10, **dadurch gekennzeichnet, daß** sich die Schürze ringsum mehr oder weniger von dem Mittelteil weg verjüngt.

12. Saugglocke nach einem der Ansprüche 7 bis 11, **dadurch gekennzeichnet, daß** der Querschnitt der Schürze im wesentlichen dreieckig oder trapezförmig ist.

13. Saugglocke nach einem der Ansprüche 7 bis 12, **dadurch gekennzeichnet, daß** das Mittelteil eben ist, und daß die Schürze einen Saum (20) hat, der in einer zu der Ebene des Mittelteils parallelen Ebene liegt.

14. Saugglocke nach einem der Ansprüche 7 bis 12, **dadurch gekennzeichnet, daß** das Mittelteil konvex gewölbt ist.

15. Saugglocke nach einem der Ansprüche 7 bis 14, **dadurch gekennzeichnet, daß** die Schürze in einem abformenden Verfahren, insbesondere gieß- oder spritzgießtechnisch hergestellt und das Mittelteil in die Schürze eingebettet ist.

16. Saugglocke nach Anspruch 15, **dadurch gekennzeichnet, daß** zumindest die nicht von der Schürze bedeckte Partie des Mittelteils transparent ist.

17. Saugglocke nach einem der Ansprüche 7 bis 16, **dadurch gekennzeichnet, daß** das Mittelteil mit einem Anschluß (28) für das Anlegen von Unterdruck versehen ist.

18. Saugglocke nach einem der Ansprüche 1 bis 17, **dadurch gekennzeichnet, daß** der daran anlegbare Unterdruck konstruktiv begrenzt ist.

19. Saugglocke nach einem der Ansprüche 1 bis 18, **dadurch gekennzeichnet, daß** eine Saugpumpe, insbesondere eine Handpumpe, daran anschließbar ist, die ein Betätigungselement vorzugsweise in Form eines elastisch komprimierbaren Balls hat.

## Claims

1. Suction cup for the nonsurgical correction of the form and/or functionality of the chest, which cup is placed in sealing contact on the anterior side of the torso and with which partial vacuum can be applied to the body, **characterized in that** the suction cup is composed at least in part of soft elastic material, is provided with inner flanks that flare toward the body when under atmospheric pressure and is applicable with lateral body-fitting portions (26) on each side of the sternum, with an upper body-fitting portion (22) on the upper region of the sternum and with a lower body-fitting portion (24) on the lower region of or below the sternum, and **in that** the deformability of the suction cup varies over its central angle and is different at least between the upper, lower or one of the lateral body-fitting portion(s) (22, 24, 26).

2. Suction cup according to claim 1, **characterized in that** the lateral body-fitting portions (26) are poorly deformable, **in that** the upper body-fitting portion (22) is moderately deformable, and **in that** the lower body-fitting portion (24) is readily deformable.

3. Suction cup according to claim 1 or 2, **characterized in that** the material of the body-fitting portions (22, 24, 26) is homogeneous and its variations in deformability are achieved by local thickness variations.

4. Suction cup according to one of claims 1 to 3, **characterized in that** the lateral body-fitting portions (26) are thick, the upper body-fitting portion (22) is thinner and the lower body-fitting portion (24) is thinnest.

5. Suction cup according to one of claims 1 to 4, **characterized in that** the body-fitting portions (22, 24, 26) are made of silicone (polyorganosiloxane).

6. Suction cup according to one of claims 1 to 5, **characterized in that** the body-fitting portions (22, 24, 26) have a hardness of between 1 Shore A and 35 Shore A.

7. Suction cup according to one of claims 1 to 6, **characterized in that** it is composed of a substantially rigid middle part and an apron of soft elastic material, especially silicone (polyorganosiloxane), sealingly mounted thereon.

8. Suction cop according to claim 7, **characterized in that** the middle part, viewed in its basic outline, has the form substantially of a circle, segment of a circle or sector of a circle.

9. Suction cup according to claim 7 or 8, **characterized in that** its middle part, viewed in its basic outline, has lateral indentations (32) whose rim contours are substantially arcs of circles.

10. Suction cup according to one of claims 7 to 9, **characterized in that** the apron becomes broader from the middle part outward, and in this case the lateral body-fitting portions (26) have the smallest span outward from the middle part, the upper body-fitting portion (22) has a lager span and the lower body-fitting portion (24) has the largest span.

11. Suction cup according to one of claims 7 to 10, **characterized in that** the apron, all around, tapers away from the middle part to a greater or lesser degree.

12. Suction cup according to one of claims 7 to 11, **characterized in that** the cross section of the apron is substantially triangular or trapezoidal.

13. Suction cup according to one of claims 7 to 12, **characterized in that** the middle part is plane, and **in that** the apron has a border (20) disposed in a plane parallel to the plane of the middle part.

14. Suction cup according to one of claims 7 to 12, **characterized in that** the middle part has convex curvature.

15. Suction cup according to one of claims 7 to 14, **characterized in that** the apron is produced by a shape-imparting method, especially casting or injection molding, and **in that** the middle part is embedded in the apron.

16. Suction cup according to claim 15, **characterized in that** at least the portion of the middle part not covered by the apron is transparent.

17. Suction cup according to one of claims 7 to 16, **characterized in that** the middle part is equipped with a port (28) for application of partial vacuum.

18. Suction cup according to one of claims 1 to 17, **characterized in that** the partial vacuum that can be applied thereto is limited by design.

19. Suction cup according to one of claims 1 to 18, **characterized in that** there can be connected thereto a suction pump, especially a hand pump, which has an actuating element, preferably in the form of an elastically compressible ball.

## Revendications

1. Cloche d'aspiration destinée à la correction non chirurgicale de la forme et/ou de la capacité de fonctionnement du thorax, laquelle est mise en appui étanche sur la face avant du tronc et par laquelle une dépression peut être appliquée sur le corps, **caractérisée en ce que** la cloche d'aspiration est réalisée au moins en partie dans un matériau élastique mou, comporte des flancs intérieurs qui s'évasent vers le corps sous l'effet de la pression atmosphérique et est apte à être mise en appui sur les côtés du sternum au moyen de parties de contact au corps latérales (26), dans la région supérieure du sternum au moyen d'une partie de contact au corps supérieure (22) et dans la région inférieure ou en dessous du sternum au moyen d'une partie de contact au corps inférieure (24), et **en ce que** la déformabilité de la cloche d'aspiration varie par l'intermédiaire de son angle au centre et est différente au moins entre la partie de contact au corps supérieure (22), la partie de contact au corps inférieure (24) ou l'une des parties de contact au corps latérales (26).

2. Cloche d'aspiration selon la revendication 1, **caractérisée en ce que** les parties de contact au corps latérales (26) sont difficilement déformables, **en ce que** la partie de contact au corps supérieure (22) est moyennement déformable, et **en ce que** la partie de contact au corps inférieure (24) est facilement déformable.

3. Cloche d'aspiration selon la revendication 1 ou 2, **caractérisée en ce que** le matériau des parties de contact au corps (22, 24, 26) est homogène et sa déformabilité différente est due à la variation locale de l'épaisseur.

4. Cloche d'aspiration selon l'une quelconque des revendications 1 à 3, **caractérisée en ce que** les parties de contact au corps latérales (26) sont épaisses, la partie de contact au corps supérieure (22) est plus mince et la partie de contact au corps inférieure (24) est la plus mince.

5. Cloche d'aspiration selon l'une quelconque des revendications 1 à 4, **caractérisée en ce que** les parties de contact au corps (22, 24, 26) sont réalisées en silicone (polyorganosiloxane).

6. Cloche d'aspiration selon l'une quelconque des revendications 1 à 5, **caractérisée en ce que** les parties de contact au corps (22, 24, 26) ont une dureté entre 1 Shore A et 35 Shore A.

7. Cloche d'aspiration selon l'une quelconque des revendications 1 à 6, **caractérisée en ce qu'**elle est formée par une partie centrale sensiblement rigide et une jupe, à attacher à celle-ci moyennant la réalisation d'une étanchéité et réalisée dans un matériau élastique mou, en particulier le silicone (polyorganosiloxane).

8. Cloche d'aspiration selon la revendication 7, **caractérisée en ce que** la partie centrale présente un tracé d'ensemble sensiblement circulaire, en forme de segment de cercle ou en forme de secteur de cercle.

9. Cloche d'aspiration selon la revendication 7 ou 8, **caractérisée en ce que**, dans le tracé d'ensemble de sa partie centrale, elle comporte des creux (32) avec un contour sensiblement en forme de cercle partiel.

10. Cloche d'aspiration selon l'une quelconque des revendications 7 à 9, **caractérisée en ce que** la jupe s'élargit à partir de la partie centrale vers l'extérieur et possède dans ce cas par rapport à la partie centrale la plus petite saillie avec les parties de contact au corps latérales (26), une saillie plus grande avec la partie de contact au corps supérieure (22) et la plus grande saillie avec la partie de contact au corps inférieure (24).

11. Cloche d'aspiration selon l'une quelconque des revendications 7 à 10, **caractérisée en ce que** la jupe se rétrécit sur le pourtour en s'écartant plus ou moins de la partie centrale.

12. Cloche d'aspiration selon l'une quelconque des revendications 7 à 11, **caractérisée en ce que** la section de la jupe est sensiblement triangulaire ou trapézoïdale.

13. Cloche d'aspiration Selon l'une quelconque des revendications 7 à 12, **caractérisée en ce que** la partie centrale est plane et **en ce que** la jupe comporte une lisière (20) qui est disposée dans un plan parallèle au plan de la partie centrale.

14. Cloche d'aspiration selon l'une quelconque des revendications 7 à 12, **caractérisée en ce que** la partie centrale présente une courbure convexe.

15. Cloche d'aspiration selon l'une quelconque des revendications 7 à 14, **caractérisée en ce que** la jupe est réalisée par un procédé de formage, en particulier par la technique de fonderie ou la technique de moulage par injection, et la partie centrale est enrobée dans la jupe.

16. Cloche d'aspiration selon la revendication 15, **caractérisée en ce que** la partie centrale est transparente au moins dans la partie non recouverte par la jupe.

17. Cloche d'aspiration selon l'une quelconque des revendications 7 à 16, **caractérisée en ce que** la partie centrale est munie d'un raccord (28) pour l'application de la dépression.

18. Cloche d'aspiration selon l'une quelconque des revendications 1 à 17, **caractérisée en ce que** la dépression apte à être appliquée est limitée par des mesures constructives.

19. Cloche d'aspiration selon l'une quelconque des revendications 1 à 18, **caractérisée en ce qu'**il est possible d'y raccorder une pompe d'aspiration, en particulier une pompe manuelle, qui comporte un élément d'actionnement, de préférence sous la forme d'un ballonnet élastiquement comprimable.
